# EUROPEAN PATENT APPLICATION

(11) **EP 1 132 755 A2**
(43) Date of publication of application: **12.09.2001**
(21) Application number: 01105898.9
(22) Date of filing: 09.03.2001
(51) Int. Cl.: G01T 1/175

(54) **Radiography apparatus**

(30) Priority: 10.03.2000 JP 2000066514
(71) Applicant: Matsushita Electric Industrial Co., Ltd., Kadoma-shi, Osaka (JP)
(72) Inventor: Yano, Shigeki, Suita-shi, Osaka (JP)
(74) Representative: Grünecker, Kinkeldey, Stockmair & Schwanhäusser Anwaltssozietät

(57) **Abstract**

A sensor unit (2) is provided with a battery (4) and a charging terminal (5) attached to the battery. The sensor unit is positioned near a battery charger (7) for recharging when the radiography apparatus is in its standby position before X-raying an object.

## Description

### BACKGROUND OF THE INVENTION

### 1.Field of the Invention

The present invention relates to radiography apparatus for clinical use.

### 2.Description of the Related Art

Conventionally, the radiography apparatus of this type generally used silver films. An X-ray image was printed on a film as a monochrome photograph.

In recent years, radiography apparatus using a variety of digital technologies have appeared on the market. One of the corresponding technologies is the computed radiography technologies whereby an X-ray image is transferred onto a special fluorescent film and the fluorescent image is read as a digital image by using a laser and stored. Another technology is one where an X-ray image is directly read by a combination of CCD and fluorescent materials as video photography.

In particular, the technology via a combination of CCD and fluorescent materials requires no radiation of laser unlike the CR technology, because a direct digital image is output as a digital image. The technology provides a very compact configuration where images are accommodated onto a size same as that of a conventional film cartridge. The technology has been already on the market as panoramic radiography apparatus.

There has emerged a problem that an image can be read out in a short period according to a combination of CCD and fluorescent materials although an external power supply, that is, a power cable is required in order to drive CCD and the peripheral circuits. This configuration also requires a signal line for sending the output signals to a PC. In this way, in case a sensor unit having a power cable and a signal line is used as panoramic radiography apparatus while rotating the sensor unit about an axis of rotation external to the sensor unit, the power cable and the signal cable get twisted around surrounding apparatus or touch the support stand for the radiography apparatus. It is thus cumbersome to route the cables off other apparatus while handling the radiography apparatus. Another trouble is that foreign noises may result from long wiring configuration as well as a break in the cable and poor contact of connectors. These problems have called for proper countermeasure.

### SUMMARY OF THE INVENTION

In order to solve the problems in the related art, this invention provides stable and high-quality digital panoramic radiography apparatus wherein routing of a power cable and a signal cable is not required and invasion of foreign noises from wiring as well as a break in the cable and poor contact of connectors are eliminated, by providing a wireless sensor unit.

In order to solve the aforementioned problems, radiography apparatus according to the first aspect of the invention comprises a sensor unit where a radiation source for radiating X-rays and a sensor for X-raying an object are housed, said sensor unit having a battery and a charging terminal, a movable stage where said radiation source and said sensor unit are arranged, a battery charger for recharging the battery for the sensor unit, and a controller for controlling the movement of said stage, wherein said charging terminal is coupled to said battery charger when said stage has returned to its standby position after X-raying the object.

Radiography apparatus according to the second aspect of the invention comprises a sensor unit where a radiation source for radiating X-rays and a sensor for X-raying an object are housed, said sensor unit having a battery and a charging terminal, a movable stage having at least a semi-circular track, said stage rotating about an axis, a battery charger for recharging the battery for the sensor unit, and a controller for controlling the rotation of said stage having at least a semi-circular track, wherein the charging terminal of said sensor unit is coupled to said battery charger when said stage has returned to its standby position after X-raying the object.

Radiography apparatus according to the third aspect of the invention comprises a sensor unit having a non-contact communication port and a controller for controlling the movement of a radiation source and the sensor unit, said controller having a non-contact communication port, said sensor unit communicating a control signal and X-raying data to/from the controller during the X-raying period.

Radiography apparatus according to the fourth aspect of the invention comprises a sensor unit having a non-contact communication port and a controller for controlling the movement of a radiation source and the sensor unit, said controller having a non-contact communication port, said sensor unit having a controller having a non-contact communication port, wherein said sensor unit transmits X-raying data to the controller when said sensor unit has returned to its standby position after X-raying the object.

### BRIEF DESCRIPTION OF THE DRAWINGS

Fig. 1 is a schematic view of radiography apparatus according to the first aspect of the invention.

Fig. 2 is a schematic view of radiography apparatus according to the first aspect of the invention while said radiography apparatus is not X-raying an object, that is, is in its standby position.

Fig. 3 is a schematic view of radiography apparatus according to the second aspect of the invention.

### DESCRIPTION OF THE PREFERRED EMBODIMENTS

According to radiography apparatus of the first aspect of the invention, a sensor unit can obtain power necessary for one round of X-raying from the power recharged in a battery, thus making unnecessary a power cable.

According to radiography apparatus of the second aspect of the invention, a sensor unit can obtain power necessary for one round of X-raying from the power recharged even in a battery in radiography apparatus that rotates about an axis, thus making unnecessary a power cable.

Radiography apparatus according to the third aspect of the invention is adapted to communicate a control signal and X-raying data between a sensor unit having a non-contact communication port and a controller having a non-contact communication port during the X-raying period.

Radiography apparatus according to the fourth aspect of the invention is adapted to transmit X-raying data from a sensor unit having a non-contact communication port to a controller having a non-contact communication port when said sensor unit has returned to its standby position after X-raying the object.

The first embodiment of the invention will be explained below.

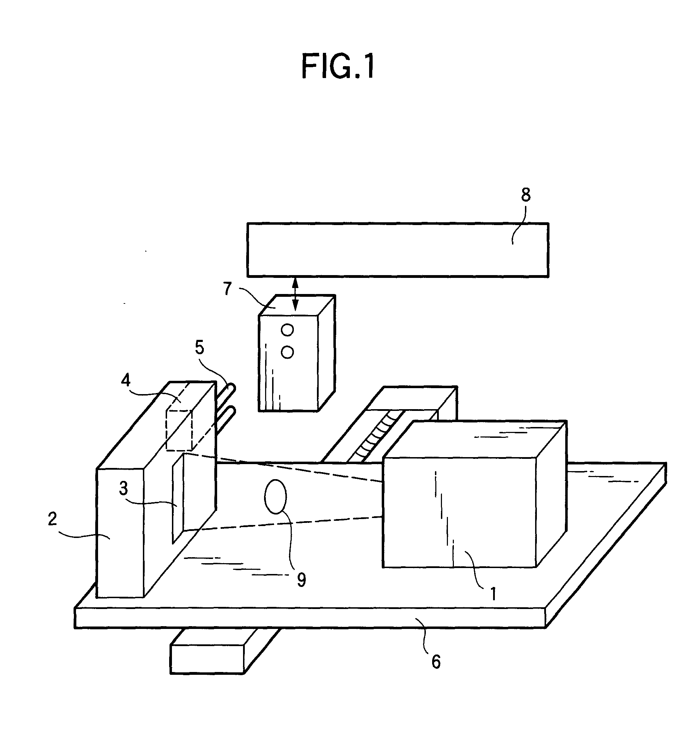

Fig. 1 is a schematic view of radiography apparatus according to the first aspect of the invention. In Fig. 1, a sign 1 represents an X-ray radiation source, a sign 2 a sensor unit, a sign 3 a sensor for detecting an X-ray that has passed through an object, a sign 4 a rechargeable battery, a sign 5 a charging terminal provided on the rechargeable battery 4, a sign 6 a stage that can be moved forward and backward, a sign 7 a battery charger for recharging the rechargeable battery 4, a sign 8 a controller for controlling the target position and distance for moving the radiation source 1 and the sensor unit 3, and a sign 9 an object.

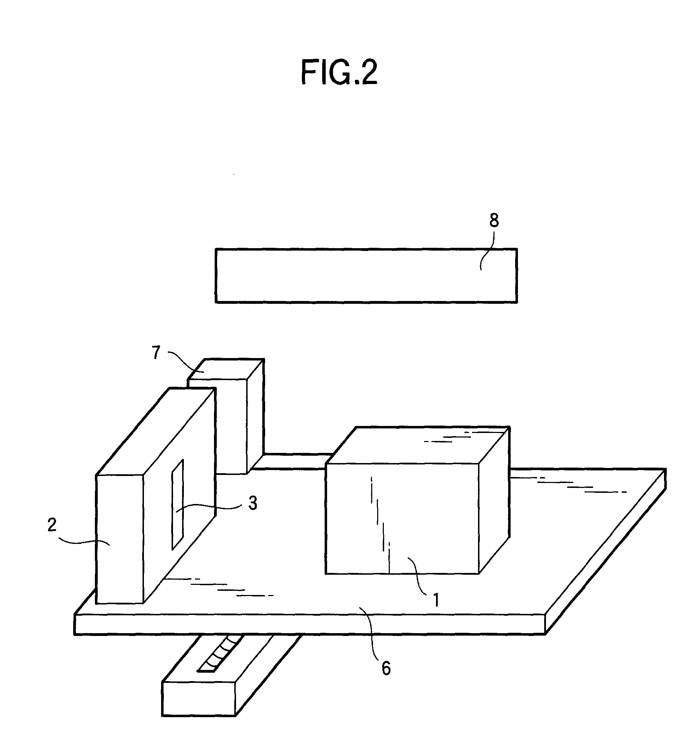

Fig. 2 is a view of radiography apparatus while said apparatus is not X-raying the object, that is, in its standby position. The stage 6 is in its standby position and the charging terminal 5 is coupled to the battery charger 7 and the battery 4 in the sensor unit 2 is being recharged by the battery charger 7 via the charging terminal 5.

In case the object 9 is X-rayed, the stage 6 is X-raying the object off its standby position, as shown in Fig. 1. The sensor unit 2 drives the sensor 3 and its peripheral circuits and generates a digital signal corresponding to the intensity of the X-ray received, via charge power from the rechargeable battery 4 inside said sensor unit 2.

While the stage 6 movable forward and backward is used as an example in this embodiment, a case where a radiation source and a sensor unit is installed on a stage having a semi-circular track as in the CT (Computerized Tomography) scan is applicable.

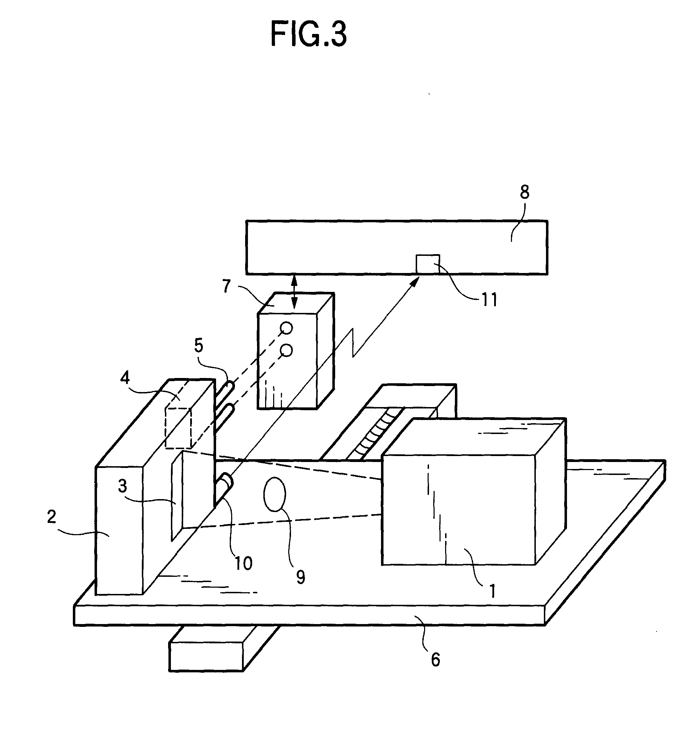

Fig. 3 is a schematic view of radiography apparatus according to another embodiment of the invention. In Fig. 3, a sign 1 represents an X-ray radiation source for radiating an X-ray on an object, a sign 2 represents a sensor unit, a sign 3 a sensor for detecting an X-ray that has passed through the object, a sign 4 a battery provided on the sensor unit 2, a sign 5 a charging terminal, a sign 6 a stage that can be moved forward and backward, a sign 7 a battery charger for recharging the rechargeable battery 4, a sign 8 a controller for controlling the target position and distance for moving the radiation source 1 and the sensor unit 3, sign 10 a non-contact communication port, and a sign 11 a non-contact receiving port provided on the controller 8 for receiving signals from a non-contact communication port.

In this embodiment also, when the radiography apparatus is not X-raying an object, that is, in its standby position, the configuration of the radiography apparatus is the same as that in Fig. 2. The stage 6 is in its standby position. The charging terminal 5 is coupled to the battery charger 7 and battery 4 in the sensor unit 2 is being recharged by the battery charger 7 via the charging terminal 5.

While the stage 6 is X-raying the object off its standby position, the sensor unit 2 drives the sensor and its peripheral circuits and generates a digital signal corresponding to the intensity of the X-ray received, via charge power from the rechargeable battery 4 inside said sensor unit 2.

Radiography apparatus according to this embodiment comprises a sensor unit having a non-contact communication port and a controller for controlling the movement of a radiation source and the sensor unit, said controller having a non-contact communication port, said sensor unit having a controller having a non-contact communication port, wherein said sensor unit transmits X-raying data to the controller when said sensor unit has returned to its standby position after X-raying the object.

Same as the first embodiment of the invention, the stage 6 may be one that can be rotated about an axis.

Any of the embodiments of the invention can be applied to panoramic radiography apparatus for dental applications, CT, and bone-density measuring apparatus using X-rays and radiography apparatus for industrial use, as well as radiography apparatus for clinical use.

As mentioned earlier, according to the invention, digital radiography apparatus for clinical use is provided with a feature whereby a sensor unit transmits X-raying data to a controller when said sensor unit has returned to its standby position after X-raying an object, via a controller having a non-contact communication port and a sensor unit having a non-contact communication port in order to provide a wireless sensor unit. Via this configuration, routing of a power cable and a signal cable is not required and invasion of foreign noises from wiring as well as break in the cable and poor contact of connectors are eliminated, thus providing stable and high-quality digital panoramic radiography apparatus. This is considerably useful in radiograph analysis in the clinical field.

## Claims

1. Radiography apparatus comprising a sensor unit where a radiation source for radiating X-rays and a sensor for X-raying an object are housed, said sensor unit having a battery and a charging terminal, a movable stage where said radiation source and said sensor unit are arranged, and a battery charger for recharging the battery for the sensor unit.

2. Radiography apparatus comprising a sensor unit where a radiation source for radiating X-rays and a sensor for X-raying an object are housed, said sensor unit having a battery and a charging terminal, a movable stage where said radiation source and said sensor unit are arranged, a battery charger for recharging the battery for the sensor unit, and a controller for controlling the rotation of said stage, wherein the charging terminal of said sensor unit is coupled to said battery charger when said stage has returned to its standby position after X-raying the object.

3. Radiography apparatus comprising a sensor unit where a radiation source for radiating X-rays and a sensor for X-raying an object are housed, said sensor unit comprising a battery and a charging terminal, a movable stage comprising at least a semi-circular track where said radiation source and said sensor unit are arranged movably, and a battery charger for recharging the battery for the sensor unit.

4. Radiography apparatus having a sensor unit where a radiation source for radiating X-rays and a sensor for X-raying an object are housed, said sensor unit having a battery and a charging terminal, a movable stage having at least a semi-circular track where said radiation source and said sensor unit are arranged, said stage rotating about an axis, and a battery charger for recharging the battery for the sensor unit.

5. Radiography apparatus according to claim 3, comprising a controller for controlling the movement of a radiation source and a sensor unit, wherein the charging terminal of said sensor unit is coupled to said battery charger when said sensor unit has returned to its standby position after X-raying the object.

6. Radiography apparatus according to claim 4, comprising a controller for controlling the rotation of a stage having at least a semi-circular track, wherein the charging terminal of said sensor unit is coupled to said battery charger when said stage has returned to its standby position after X-raying the object.
